# EUROPEAN PATENT APPLICATION

(11) **EP 3 498 853 A1**
(43) Date of publication of application: **19.06.2019**
(21) Application number: 17207318.1
(22) Date of filing: 14.12.2017
(51) Int. Cl.: C12P 13/08, C12R 1/15

(54) **METHOD FOR THE FERMENTATIVE PRODUCTION OF L-LYSINE**

(71) Applicant: Evonik Degussa GmbH, 45128 Essen (DE)
(72) Inventor: VOSS, Kornelia, 33790 Halle (DE); THIERBACH, Georg, 33613 Bielefeld (DE); BEKEL, Thomas, 33790 Hallle (Westf.) (DE)

(57) **Abstract**

The present invention makes available novel L-lysine excreting bacteria of the species *Corynebacterium glutamicum,* having the ability to excrete L-lysine, containing in their chromosome a polynucleotide encoding a polypeptide having the activity of a malate:quinone reductase wherein the amino acid at position 228 of the amino acid sequence of the polypeptide contains any proteinogenic amino acid different from valine and a method for producing L-lysine using such bacteria.

## Description

L-lysine is used in human medicine, in the pharmaceutical industry, in the food industry and particularly in nutrition of animals.

It is known that L-lysine is produced by fermentation of strains of the species *Corynebacterium glutamicum.* Because of the great economic importance, work is continually being done on improving the production methods. Improvements may relate to the fermentation technology such as e. g. stirring and supplying oxygen, or to the composition of the nutrient media e.g. the sugar concentration during fermentation, or to the processing of the fermentation broth to a suitable product form by e. g. drying and granulating the fermentation broth or ion exchange chromatography or may relate to the intrinsic performance properties of the microorganism itself.

The methods used for improving the performance properties of these microorganisms are those of mutagenesis, selection and screening of mutants. The strains obtained in this way are resistant to anti-metabolites or are auxotrophic for metabolites of regulatory importance, and produce L-lysine. A well-known anti-metabolite is the L-lysine analogue S-(2-aminoethyl)-L-cysteine (see e.g. Tosaka et al.: Agricultural and Biological Chemistry 42(4), 745-752, (1978)).

Methods of recombinant DNA technology have likewise been used for a number of years for improvement of L-lysine-producing strains of the species *Corynebacterium glutamicum,* by modifying, i.e. enhancing or attenuating, individual L-lysine biosynthesis genes and investigating the effect on L-lysine production.

The nucleotide sequences of the chromosomes of various bacteria or strains resp. of the species *Corynebacterium glutamicum,* and their analysis have been disclosed. This information is available at publicly accessible data bases and may be used for strain development purposes. One such data base is the GenBank data base of the NCBI (National Center for Biotechnology Information, U.S. National Library of Medicine 8600 Rockville Pike, Bethesda MD, 20894 USA).

During the annotation procedure for a sequenced chromosome of an organism identified structures such as e. g. genes or coding sequences are furnished with a unique identifier called locus_tag by the supplier of the information to the data base.

The nucleotide sequence of the *Corynebacterium glutamicum* ATCC13032 chromosome and its analysis were described by Ikeda and Nakagawa (Applied Microbiology and Biotechnology 62, 99-109(2003)) and in EP1108790A2. The information is available at the NCBI under accession number NC_003450. In the chromosome sequence disclosed under accession number NC_003450 locus_tag NCgl1926 identifies a nucleotide sequence coding for a malate:quinone oxidoreductase. The amino acid sequence of the polypeptide is available under the identifier NP_60120.

The nucleotide sequence of the *Corynebacterium glutamicum* ATCC13032 chromosome and its analysis were independently described by Kalinowski et al. (Journal of Biotechnology 104 (1-3), 5-25 (2003)). The information is available at the NCBI under accession number NC_006958. Locus_tag CGTRNA_RS09800 identifies a nucleotide sequence coding for a malate:quinone oxidoreductase. The old_locus_tag designation cg2192 is also used in the art. The amino acid sequence of the polypeptide is available under the identifier WP_011014814.

The nucleotide sequences of locus_tag NCgl1926 and CGTRNA_RS09800 are identical.

Lv et al. (Journal of Bacteriology 194(3), 742-743 (2012) describe the sequencing of the chromosome of *Corynebacterium glutamicum* ATCC14067, a strain formerly referred to as *Brevibacterium flavum.* The information is available at the NCBI under accession number AGQQ02000001 and AGQQ02000002. The nucleotide sequence of the chromosome of *Corynebacterium glutamicum* ATCC13869, a strain formerly referred to as *Brevibacterium lactofermentum,* and its analysis were disclosed by Chen et al. at the NCBI under accession number NZ_CP016335.

Malate:quinone oxidoreductase is a membrane associated enzyme catalyzing the oxidation of malate to oxaloacetate with quinones serving as electron acceptors. The biochemical and genetic characterization of the enzyme of *Corynebacterium glutamicum* and its function were described by Molenaar et al. (European Journal of 254, 395-403 (1998)) and Molenaar et al. (Journal of Bacteriology 182(24), 6884-6891 (2000)). Molenaar et al. referred to the enzyme as EC 1.1.99.16. According to current IUBMB Enzyme Nomenclature the enzyme is given the systematic designation EC 1.1.5.4 with malate dehydrogenase (quinone) as accepted name and (S)-malate:quinone oxidoreductase as systematic name. The art uses the abbreviation Mqo for the enzyme or for the polypeptide resp.. The gene encoding the Mqo polypeptide is called *mqo.* Information concerning transcription signals, e.g. -10 region of a promoter, the 5'-untranslated leader sequence (5'-UTR), and translation signals, e.g. ribosome binding site (RBS), of the *mqo* gene (old_locus_tag cg2192) can be found in Pfeifer-Sancar et al. (BMC Genomics 14:888 (2013)) and Han et al. (Journal of Molecular Microbiology and Biotechnology 15:264-276, 2008).

US20020028490A1 describes the favorable effect of overexpression of malate:quinone oxidoreductase on L-lysine and L-threonine production by *Corynebacterium glutamicum.*

WO02086137A2 describes the favorable effect of attenuating or eliminating the malate:quinone oxidoreductase activity on L-lysine production by *Corynebacterium glutamicum.*

WO03029457A1 likewise describes the favorable effect of attenuating or eliminating the malate:quinone oxidoreductase activity on L-lysine production by *Corynebacterium glutamicum.*

US20060166338A1 describes the favorable effect of an amino acid exchange at position 111 of the amino acid sequence of the malate:quinone oxidoreductase on L-lysine production in *Corynebacterium glutamicum.*

Object of the present invention is to provide new measures for the fermentative production of L-lysine by bacteria of the species *Corynebacterium glutamicum.*

To achieve the object outlined above the present invention makes available novel L-lysine excreting bacteria of the species *Corynebacterium glutamicum,* having the ability to excrete L-lysine, containing in their chromosome a polynucleotide encoding a polypeptide having the activity of a malate:quinone reductase wherein the amino acid at position 228 of the amino acid sequence of the polypeptide contains any proteinogenic amino acid different from valine.

The present invention further makes available methods for producing said L-lysine by using said bacteria in a fermentative process and for the manufacturing of a product containing said L-lysine from the fermentation broth.

The objects underlying the present invention were solved by the means as written in the claims.

Accordingly the present invention provides the following:
A bacterium of the species *Corynebacterium glutamicum,* having the ability to excrete L-lysine, containing in its chromosome a polynucleotide encoding a polypeptide having the activity of a malate:quinone oxidoreductase and comprising the amino acid sequence of SEQ ID NO:2, wherein the amino acid valine at position 228 is substituted by a different proteinogenic amino acid, preferably by aspartic acid or glutamic acid, particular preferred by aspartic acid.

It was found that the bacteria modified according to the invention excreted L-lysine, into a suitable medium under suitable fermentation conditions in an increased manner with respect to one or more parameters selected from product concentration (i.e. amount of L-lysine produced per volume, or mass unit resp., of medium/fermentation broth (e.g. g/l or g/kg)), product yield (i.e. amount of L-lysine produced per carbon source consumed (e.g. g/g or kg/kg)), product formation rate (i.e. amount of L-lysine produced per volume, or mass unit resp., of medium/fermentation broth and unit of time (e.g. g/l x h or g/kg x h)), and specific product formation rate (i.e. amount of L-lysine produced per unit of time and mass unit of the producer (e.g. g/h x g dry mass)) as compared to the unmodified bacterium.

It is obvious that a higher product concentration facilitates product manufacturing e. g. purification and isolation. An increased product yield reduces the amount of raw material required. An increased product formation rate reduces the time required for a fermentation run thus increasing the availability of a given fermenter.

The bacteria according to the invention thus contribute to the improvement of technical and economic aspects of the manufacturing of L-lysine or L-lysine containing products.

In a further embodiment the bacterium according to the invention contains in its chromosome a polynucleotide encoding an amino acid sequence of a polypeptide having malate:quinone oxidoreductase activity comprising the amino acid sequence according to SEQ ID NO:2, wherein the amino acid valine at position 228 of the encoded amino acid sequence of SEQ ID NO:2 is substituted by aspartic acid, and comprising the nucleotide sequence of positions 1001 to 2500 of SEQ ID NO:1 the nucleobase at position 1683 being adenine or comprising the nucleotide sequence of positions 1001 to 2500 of SEQ ID NO:3.

In a further embodiment the bacterium according to the invention contains in its chromosome a polynucleotide which is coding for an amino acid sequence of a polypeptide having malate:quinone oxidoreductase activity comprising the amino acid sequence according to SEQ ID NO:2, wherein the amino acid valine at position 228 of the encoded amino acid sequence of SEQ ID NO:2 is substituted by a different proteinogenic amino acid, preferably by aspartic acid or glutamic acid, particularly preferred by aspartic acid, and wherein the 5'-end of the coding sequence of this polynucleotide is directly connected to the 3'-end of the polynucleotide comprising the nucleotide sequence from positions 781 to 1000 of SEQ ID NO:1 or of SEQ ID NO:3. In particular, the bacterium of the species *Corynebacterium glutamicum* according to the present invention contains in its chromosome a polynucleotide encoding the polypeptide comprising the amino acid sequence according to SEQ ID NO:2 the amino acid at position 228 being aspartic acid and having the activity of a malate:quinone oxidoreductase which comprises the nucleotide sequence from positions 781 to 2500 of SEQ ID NO:3 or the nucleotide sequence from positions 781 to 2500 of SEQ ID NO:1 the nucleobase at position 1683 being adenine. In other words, the bacterium according to the present invention may contain in its chromosome a polynucleotide encoding said amino acid sequence which comprises the nucleotide sequence from positions 781 to 2500 of SEQ ID NO:1 whereby the nucleobase at position 1683 is adenine or the nucleotide sequence from positions 781 to 2500 of SEQ ID NO:3.

The term L-lysine, where mentioned herein, in particular in the context of product formation, also comprises their ionic forms and salts, for example L-lysine mono hydrochloride or L-lysine sulfate.

For practicing the present invention bacteria of the species *Corynebacterium glutamicum* are used. A description of the genus *Corynebacterium* and the species *Corynebacterium glutamicum* comprised by this genus can be found in the article *"*Corynebacterium" by K. A. Bernard and G. Funke in Bergey's Manual of Systematics of Archaea and Bacteria (Bergey's Manual Trust, 2012).

Suitable strains of *Corynebacterium glutamicum* are wild strains of this species for example strains ATCC13032, ATCC14067 and ATCC13869, and L-lysine excreting strains obtained from these wild strains.

Strain ATCC13032 (also available as DSM20300) is the taxonomic type strain of the species *Corynebacterium glutamicum.* Strain ATCC14067 (also available as DSM20411) is also known under the outdated designation *Brevibacterium flavum.* Strain ATCC13869 (also available as DSM1412) is also known under the outdated designation *Brevibacterium lactofermentum.* A taxonomic study of this group of bacteria based on DNA-DNA hybridization was done by Liebl et al. (International Journal of Systematic Bacteriology 41(2), 255-260, 1991). A comparative analysis of various strains of the species *Corynebacterium glutamicum* based on genome sequence analysis was provided by Yang and Yang (BMC Genomics 18(1):940).

A multitude of L-lysine excreting strains of the genus *Corynebacterium,* in particular of the species *Corynebacterium glutamicum* were obtained in the art during the past decades starting from strains such as ATCC13032, ATCC14067, ATCC13869 and the like. They were obtained as a result of strain development programs using inter alia methods like classical mutagenesis, selection for antimetabolite resistance as well as amplification and promotor modification of genes of the biosynthetic pathway of the L-amino acid in question by genetic engineering methods. Summaries may be found in L. Eggeling and M. Bott (Handbook of Corynebacterium glutamicum, CRC Press, 2005) or H. Yukawa and M. Inui (Corynebacterium glutamicum Biology and Biotechnology, Springer Verlag, 2013).

L-lysine excreting strains of the species *Corynebacterium glutamicum* are widely known in the art and can be used for the purpose of the present invention. For example Blombach et al. (Applied and Environmental Microbiology 75(2), 419-427, 2009) describe strain DM1933, which is deposited under accession DSM25442 according to the Budapest treaty. Strain DM1933 was obtained from ATCC13032 by several steps of strain development. Furthermore L-lysine excreting *Corynebacterium glutamicum* strain DM2031, deposited according to the Budapest Treaty as DSM32514 may be used. Strain DM2031 is a further developed derivative of DM1933 having enhanced L-lysine excretion ability. Other L-lysine excreting *Corynebacterium glutamicum* strains are e. g. described in WO2008033001A1 and EP0841395A1.

L-lysine excreting strains of the species *Corynebacterium glutamicum* typically contain a polynucleotide coding for a feedback resistant aspartokinase polypeptide variant. A feedback resistant aspartokinase polypeptide variant means an aspartokinase which is less sensitive, or desensitized resp., to inhibition by mixtures of L-lysine and L-threonine, e.g. 10 mM each, or mixtures of the L-lysine analogue S-(2-aminoethyl)-L-cysteine and L-threonine, e.g. 50 mM S-(2-aminoethyl)-L-cysteine and 10 mM L-threonine, when compared to the wild form of the enzyme, which is contained in wild strains like for example ATCC13032, ATCC14067 and ATCC13869. The EC number for aspartokinase is EC 2.7.2.4. Descriptions of polynucleotides of *Corynebacterium glutamicum* encoding a feedback resistant aspartokinase polypeptide variant are for example given in US5688671, US6844176 and US6893848. A summarizing list can be found inter alia in US20090311758A1. The symbol used in the art for a gene coding for an aspartokinase polypeptide is *lysC.* In case the gene codes for a feedback resistant polypeptide variant the art typically uses symbols like *lysC^{fbr}* with fbr indicating feedback resistance.

Accordingly said L-lysine excreting strains of the species *Corynebacterium glutamicum* used for the measures of the present invention preferably contain at least one copy of a polynucleotide coding for a feedback resistant aspartokinase polypeptide.

SEQ ID NO:5 shows the nucleotide sequence of the coding sequence of the aspartokinase polypeptide of strain ATCC13032 and SEQ ID NO:6 the amino acid sequence of the encoded polypeptide. It is known in the art (see US6893848) that exchange of the amino acid Thr at position 311 of SEQ ID NO:6 for Ile imparts the enzyme feedback resistance to inhibition by mixtures of L-lysine and L-threonine.

Accordingly it is preferred that the amino acid sequence of said feedback resistant aspartokinase polypeptide comprises the amino acid sequence of SEQ ID NO:6 containing isoleucine at position 311.

Said amino exchange can be achieved by exchanging the nucleobase cytosine (c) at position 932 of SEQ ID NO:5 to give thymine (t). The acc codon for threonine is thus altered to the atc codon for isoleucine.

It is further known in the art that exchange of the gtg start codon of the coding sequence for the aspartokinase polypeptide for atg enhances expression of the polypeptide (see e.g. EP2796555A2).

Accordingly it is preferred that the sequence coding for a feedback resistant aspartokinase polypeptide begins with an atg start codon.

Summaries concerning the breeding of L-lysine excreting strains of *Corynebacterium glutamicum* may be found inter alia in L. Eggeling and M. Bott (Handbook of Corynebacterium glutamicum, CRC Press, 2005), V. F. Wendisch (Amino Acid Biosynthesis - Pathways, Regulation and Metabolic Engineering, Springer Verlag, 2007), H. Yukawa and M. Inui (Corynebacterium glutamicum Biology and Biotechnolgy, Springer Verlag, 2013) or A. Yokota and M. Ikeda (Amino Acid Fermentation, Springer Verlag, 2017), and Eggeling and Bott (Applied Microbiology and Biotechnology 99 (9), 3387-3394, 2015).

In case a wild strain, e.g. ATCC13032, ATCC13869 or ATCC14067, is in a first step subjected to the measures of the present invention the resulting strain is in a second step subjected to a strain development program targeted at L-lysine in order to obtain a bacterium according to the present invention.

The term DSM denotes the depository Deutsche Sammlung für Mikroorganismen und Zellkulturen located in Braunschweig, Germany. The term ATCC denotes the depository American Type Culture Collection located in Manasass, Virginia, US.

Details regarding the biochemistry and chemical structure of polynucleotides and polypeptides as present in living things such as bacteria like *Corynebacterium glutamicum* or *Escherichia coli,* for example, can be found inter alia in the text book "Biochemie" by Berg et al. (Spektrum Akademischer Verlag Heidelberg, Berlin, Germany, 2003; ISBN 3-8274-1303-6).

Polynucleotides consisting of deoxyribonucleotide monomers containing the nucleobases or bases resp. adenine (a), guanine (g), cytosine (c) and thymine (t) are referred to as deoxyribopolynucleotides or deoxyribonucleic acid (DNA). Polynucleotides consisting of ribonucleotide monomers containing the nucleobases or bases resp. adenine (a), guanine (g), cytosine (c) and uracil (u) are referred to as ribo-polynucleotides or ribonucleic acid (RNA). The monomers in said polynucleotides are covalently linked to one another by a 3',5'-phosphodiester bond. By convention single strand polynucleotides are written from 5'- to 3'-direction. Accordingly a polynucleotide has a 5'-end and 3'-end. The order of the nucleotide monomers in the polynucleotide is commonly referred to as nucleotide sequence. Accordingly a polynucleotide is characterized by its nucleotide sequence. For the purpose of this invention deoxyribopolynucleotides are preferred. In bacteria, for example *Corynebacterium glutamicum* or *Escherichia coli,* the DNA is typically present in double stranded form. Accordingly the length of a DNA molecule is typically given in base pairs (bp). The nucleotide sequence coding for a specific polypeptide is called coding sequence (cds). A triplett of nucleotides specifying an amino acid or a stop signal for translation is called a codon. The codons specifying different amino acids are well known in the art.

A gene from a chemical point of view is a polynucleotide, preferably a deoxyribopolynucleotide.

DNA can be synthesized *de novo* by the phoshoramidite method (McBride and Caruthers: Tetrahedon Letters 24(3) 245- 248 (1983)). Summaries concerning *de novo* DNA synthesis may be found in the review article of Kosuri and Chruch (Nature Methods 11(5), 499 - 507 (2014)) or in the article of Graf et al. in the book "Systems Biology and Synthetic Biology" by P. Fu and S. Panke (John Wiley, US, 2009, pp 411-438).

The term gene refers to a polynucleotide comprising a nucleotide sequence coding for a specific polypeptide (coding sequence) and the adjoining stop codon. In a broader sense the term includes regulatory sequences preceding and following the coding sequence. The preceding sequence is located at the 5'-end of the coding sequence and is also referred to as upstream sequence. A promotor is an example of a regulatory sequence located 5' to the coding sequence. The sequence following the coding sequence is located at its 3'-end and also referred to as downstream sequence. In the context of the present invention said regulatory sequence, comprising inter alia the promotor and the ribosome binding site, of the mqo gene preceding its coding sequence is located from position 781 to 1000 of SEQ ID NO:1 or SEQ ID NO:3. A transcriptional terminator is an example of a regulatory sequence located 3' to the coding sequence.

Polypeptides consist of L-amino acid monomers joined by peptide bonds. For abbreviation of L-amino acids the one letter code and three letter code of IUPAC (International Union of Pure and Applied Chemistry) is used. Due to the nature of polypeptide biosynthesis polypeptides have an amino terminal end and a carboxyl terminal end also referred to as N-terminal end and C-terminal end. The order of the L-amino acids or L-amino acid residues resp. in the polypeptide is commonly referred to as amino acid sequence. Polypeptides are also referred to as proteins.
Further it is known in the art that the start codon or initiation codon resp. gtg of a coding sequence as well as atg encodes the amino acid methionine. It is known in the art that the N-terminal amino acid methionine of an encoded polypeptide may be removed by an aminopeptidase during or after translation (Jocelyn E. Krebs, Elliott S. Goldstein and Stephan T. Kilpatrick: Lewin's Genes X, Jones and Bartlett Publishers, US, 2011).

Proteinogenic L-amino acids are to be understood to mean the L-amino acids present in natural proteins, that is proteins of microorganisms, plants, animals and humans. Proteinogenic L-amino acids comprise L-aspartic acid, L-asparagine, L-threonine, L-serine, L-glutamic acid, L-glutamine, L-glycine, L-alanine, L-cysteine, L-valine, L-methionine, L-isoleucine, L-leucine, L-tyrosine, L-phenylalanine, L-histidine, L-lysine, L-tryptophan, L-arginine, L-proline and in some cases L-selenocysteine and L-pyrrolysine. It is common in the ass to refer to these proteinogenic L-amino acids without hinting to the L configuration at the α carbon atom of the L-amino acid; e.g. L-valine may simply called valine, L-aspartic acid may simply be called aspartic acid etc..

Teachings and information concerning the handling of and experimental work with polynucleotides may be found inter alia in the handbook of J. Sambrook et al. (Molecular Cloning, A Laboratory Manual, Cold Spring Harbor Laboratory Press, 1989), the textbook of C. R. Newton and A. Graham (PCR, Spektrum Akademischer Verlag, 1994) and the handbook of D. Rickwood and B. D. Hames (Gel electrophoresis of nucleic acids, a practical approach, IRL Press, 1982).

For sequence analysis of polynucleotides and polypeptides, e.g. sequence alignments the Clustal W program (Larkin et al.: Clustal W and Clustal X version 2.0. In: Bioinformatics 23, 2947-2948 (2007)) or public software such as the CLC Genomics Workbench (Qiagen, Hilden, Germany) or the program MUSCLE provided by the European Bioinformatics Institute (EMBL-EBI, Hinxton, UK) may be used.

*Corynebacterium glutamicum,* in particular strain ATCC13032 and L-lysine excreting strains obtained therefrom during a strain development program, contain in their chromosome among others a gene encoding a polypeptide having the activity of a malate:quinone oxidoreductase and comprising the amino acid sequence of SEQ ID NO:2. The coding sequence is shown SEQ ID NO:1 positions 1001 to 2500. The coding sequence may contain silent mutations which do not alter the amino acid sequence of the Mqo polypeptide. For example the amino acid Phe at position 410 of the amino acid sequence shown in SEQ ID NO:2 or SEQ ID NO:4 may be specified by the codon ttc or by the codon ttt (see positions 2228 to 2230, in particular position 2230, of SEQ ID NO:1 or SEQ ID NO:3). This context is also known as degeneracy of the genetic code in the art.

During the work for the present invention it was found that modifying L-lysine excreting bacteria of the species *Corynebacterium glutamicum* by exchanging the amino acid valine at position 228 of the encoded amino acid sequence of the Mqo polypeptide shown in SEQ ID NO:2 for a different proteinogenic, preferably aspartic acid or glutamic acid, particular preferred aspartic acid, increased their ability to excrete L-lysine as compared to the unmodified bacterium.

The skilled artisan is aware of a number of methods of mutagenesis how to achieve said modification in the *Corynebacterium glutamicum.*

A mutant bacterium according to the invention can be obtained by classical *in vivo* mutagenesis executed with cell populations of strains of *Corynebacterium glutamicum* using mutagenic substances, e.g. N-methyl-N'-nitro-N-nitrosoguanidine, or ultra violet light. The nucleotide sequence comprising the site of mutagenesis within the *mqo* gene can be amplified by PCR using primers selected from SEQ ID NO:1 or SEQ ID NO:3. By sequencing the PCR product the desired mutants are identified. Details concerning this approach can be found inter alia in US7754446.

A further method is the CRISPR-Cpf1 assisted genome editing described by Jiang et al. (Nature Communications 8: 15179 DOI: 10; 1038/ncomms15179).

Another common method of mutating genes of *Corynebacterium glutamicum* is the method of gene replacement described by Schwarzer and Pühler (Bio/Technology 9, 84-87 (1991)) and further elaborated by Schafer et al. (Gene 145, 69-73 (1994)).

Peters-Wendisch et al. (Microbiology 144, 915-927 (1998)) used the gene replacement method to inactivate the *pyc* gene of *Corynebacterium glutamicum* encoding pyruvate carboxylase. Schafer et al. used the method to incorporate a deletion into the *hom-thrB* gene region of *Corynebacterium glutamicum.* In EP1094111 the method was used to incorporate a deletion into the *pck* gene of *Corynebacterium glutamicum* encoding phosphoenol pyruvate carboxykinase. In US7585650 the method was applied to the *zwf* gene to realize an amino acid exchange at position 321 of the amino acid sequence of the Zwf sub-unit of the glucose 6-phosphate dehydrogenase. In US7754446 the method was applied to the *rel* gene to realize an amino acid exchange at position 38 of the amino acid sequence of the GTP- pyrophosphate kinase polypeptide.

In the gene replacement method, a mutation, for example, a deletion, insertion or substitution of at least one nucleobase, is provided by an isolated polynucleotide comprising the nucleotide sequence of the gene in question or a part thereof containing the mutation.

In the context of the present invention the nucleotide sequence of the gene in question is the *mqo* gene.

In the context of the present invention the mutation is a substitution of at least one nucleobase located in the codon specifying the amino acid valine at position 228 of the encoded amino acid sequence (see SEQ ID NO:1 and SEQ ID NO:3) of the Mqo polypeptide.

As a consequence of said mutation the codon specifies a proteinogenic amino acid different from valine, preferably aspartic acid or glutamic acid, particular preferred aspartic acid. The codons specifying aspartic acid are gat or gac. The codon gac is preferred.

The codon for the amino acid at position 228 has the position from 1682 to 1684 in SEQ ID NO:1 or SEQ ID NO:3. The nucleotide sequence from position 1682 to 1684, in particular the nucleotide at position 1683, may also be referred to as site of mutation.

The mutated nucleotide sequence of the gene in question or a part thereof containing the mutation comprises i) a nucleotide sequence at the 5'-end of the site of mutation, which is also referred to as 5'-flanking sequence or upstream sequence in the art, ii) a nucleotide sequence at the 3'-end of the site of mutation, which is also referred to as 3'-flanking sequence or downstream sequence in the art, and iii) the nucleotide sequence of the site of mutation between i) and ii).

Said 5'-flanking sequence and 3'-flanking sequence required for homologous recombination typically have a length of at least 200 bp, at least 400 bp, at least 600 bp or at least 800 bp. The maximum length typically is 1000 bp, 1500 bp or 2000 bp.

An example of a mutated nucleotide sequence in the context of the present invention comprises the nucleotide sequence shown in SEQ ID NO:1 from positions 1083 to 2283 with adenine at position 1683. Another example of a mutated nucleotide sequence in the context of the present invention comprises the nucleotide sequence shown in SEQ ID NO:3 from positions 1083 to 2283.

A further example of a mutated nucleotide sequence in the context of the present invention comprises the nucleotide sequence shown in SEQ ID NO:7. The nucleotide sequence of SEQ ID NO:7 corresponds to SEQ ID NO:3 from positions 882 to 2484. SEQ ID NO:7 contains a part of the coding sequence of the mutated *mqo* gene. Said part of the coding sequence present in SEQ ID NO:7 encodes the N'-terminal end of the Mqo polypeptide i.e. the amino acids from positions 1 to 494. Said part of the coding sequence lacks the sequence coding for the c-terminal end of the Mqo polypeptide (see SEQ ID NO:8).

The 5'-flanking sequence comprises the nucleotide sequence from positions 1 to 801 of SEQ ID NO:7. The 3'-flanking sequence consists of the nucleotide sequence from positions 803 to 1603 of SEQ ID NO:7. The site of mutation is at position 802.

The mutated nucleotide sequence provided is cloned into a plasmid vector that is not capable of autonomous replication in *Corynebacterium glutamicum.* Said plasmid vector comprising said mutated nucleotide sequence is subsequently transferred into the desired strain of *Corynebacterium glutamicum* by transformation or conjugation. After two events of homologous recombination comprising a recombination event within the 5'-flanking sequence provided by the plasmid vector with the homologous sequence of the *Corynebacterium glutamicum* chromosome and a recombination event within the 3'-flanking sequence provided by the plasmid vector with the homologous sequence of the *Corynebacterium glutamicum* chromosome, one effecting integration and one effecting excision of said plasmid vector, the mutation is incorporated in the *Corynebacterium glutamicum* chromosome. Thus the nucleotide sequence of the gene in question contained in the chromosome of said desired strain is replaced by the mutated nucleotide sequence.

An event of homologous recombination may also be referred to as crossing over.

It is preferred that the L-lysine excreting *Corynebacterium glutamicum* strains of the present invention have the ability to excrete ≥ 0,25 g/l, preferably ≥ 0,5 g/l, particularly preferred ≥ 1,0 g/l of L-lysine in a suitable medium under suitable conditions.

The invention further provides a fermentative process for producing L-lysine, using the *Corynebacterium glutamicum* of the present invention.

In a fermentative process according to the invention a *Corynebacterium glutamicum* modified in accordance with the present invention and having the ability to excrete L-lysine is cultivated in a suitable medium under suitable conditions. Due to said ability to excrete said L-lysine the concentration of the L-lysine increases and accumulates in the medium during the fermentative process and the L-lysine is thus produced.

The fermentative process may be discontinuous process like a batch process or a fed batch process or a continuous process. A summary concerning the general nature of fermentation processes is available in the textbook by H. Chmiel (Bioprozesstechnik, Spektrum Akademischer Verlag, 2011), in the textbook of C. Ratledge and B. Kristiansen (Basic Biotechnology, Cambridge University Press,2006) or in the textbook of V.C. Hass and R. Pörtner (Praxis der Bioprozesstechnik Spektrum Akademischer Verlag, 2011).

A suitable medium used for the production of L-lysine by a fermentative process contains a carbon source, a nitrogen source, a phosphorus source, inorganic ions and other organic compounds as required.

Suitable carbon sources include glucose, fructose, sucrose as well as the corresponding raw materials like starch hydrolysate, molasses or high fructose corn syrup.

As nitrogen source organic nitrogen-containing compounds such as peptones, meat extract, soy bean hydrolysates or urea, or inorganic compounds such as ammonium sulphate, ammonium chloride, ammonium phosphate, ammonium carbonate, ammonium nitrate, ammonium gas or aqueous ammonia can be used.
As phosphorus source, phosphoric acid, potassium dihydrogen phosphate or dipotassium hydrogen phosphate or the corresponding sodium-containing salts can be used.

Inorganic ions like potassium, sodium, magnesium, calcium, iron and further trace elements etc. are supplied as salts of sulfuric acid, phosphoric acid or hydrochloric acid.

Other organic compounds means essential growth factors like vitamins e. g. thiamine or biotin or L-amino acids e. g. L-homoserine.

The media components may be added to the culture in form of a single batch or be fed in during the cultivation in a suitable manner.

During the fermentative process the pH of the culture can be controlled by employing basic compounds such as sodium hydroxide, potassium hydroxide, ammonia or aqueous ammonia, or acidic compounds such as phosphoric acid or sulphuric acid in a suitable manner. The pH is generally adjusted to a value of from 6.0 to 8.5, preferably 6.5 to 8.0. To control foaming, it is possible to employ antifoam agents such as, for example, fatty acid polyglycol esters. To maintain the stability of plasmids, it is possible to add to the medium suitable selective substances such as, for example, antibiotics. The fermentative process is preferably carried out under aerobic conditions. In order to maintain these conditions, oxygen or oxygen-containing gas mixtures such as, for example air are introduced into the culture. The fermentative process is carried out, where appropriate, at elevated pressure, for example at an elevated pressure of 0.03 to 0.2 MPa. The temperature of the culture is normally from 25 °C to 40 °C, preferably from 30 °C to 37 °C. In a discontinuous process, the cultivation is continued until an amount of the L-lysine sufficient for being recovered has been formed. The cultivation is then completed. This aim is normally achieved within 10 hours to 160 hours. In continuous processes, longer cultivation times are possible.

Examples of suitable media and culture conditions can be found inter alia in L. Eggeling and M. Bott (Handbook of Corynebacterium glutamicum, CRC Press, 2005) and the patent documents US5770409, US5990350, US 5275940, US5763230 and US6025169.

Thus the fermentative process results in a fermentation broth which contains the desired L-lysine.

Accordingly a method for the fermentative production of L-lysine is provided comprising the steps of
a) cultivating a *Corynebacterium glutamicum* of the present invention in a suitable medium under suitable conditions, and
b) accumulating said L-lysine in the medium to produce an L-lysine containing fermentation broth.

A product containing the L-lysine is then recovered in liquid or solid from the fermentation broth.

A "fermentation broth" means a medium in which a *Corynebacterium glutamicum* of the invention has been cultivated for a certain time and under certain conditions.

When the fermentative process is completed, the resulting fermentation broth accordingly comprises:
a) the biomass (cell mass) of the *Corynebacterium glutamicum* of the invention, said biomass having been produced due to propagation of the cells of said *Corynebacterium glutamicum,*
b) the desired L-lysine accumulated during the fermentative process,
c) the organic by-products accumulated during the fermentative process, and
d) the components of the medium employed which have not been consumed in the fermentative process.

The organic by-products include compounds which may be formed by the *Corynebacterium glutamicum* of the invention during the fermentative process in addition to production of the L-lysine.

The fermentation broth is removed from the culture vessel or fermentation tank, collected where appropriate, and used for providing a product containing the L-lysine, in liquid or solid form. The expression "recovering the L-lysine-containing product" is also used for this. In the simplest case, the L-lysine -containing fermentation broth itself, which has been removed from the fermentation tank, constitutes the recovered product.

The fermentation broth can subsequently be subjected to one or more of the measures selected from the group consisting of:
a) partial (>0% to <80%) to complete (100%) or virtually complete ( ≥ 80%, ≥ 90%, ≥ 95%, ≥ 96%, ≥ 97%, ≥ 98%, ≥ 99%) removal of the water,
b) partial (>0% to <80%) to complete (100%) or virtually complete (≥ 80%, ≥ 90%, ≥ 95%, ≥ 96%, ≥ 97%, ≥ 98%, ≥ 99%) removal of the biomass, the latter being optionally inactivated before removal,
c) partial (>0% to <80%) to complete (100%) or virtually complete (≥ 80%, ≥ 90%, ≥ 95%, ≥ 96%, ≥ 97%, ≥ 98%, ≥ 99%, ≥ 99.3%, ≥ 99.7%) removal of the organic by-products formed during the fermentative process, and
d) partial (>0% to <80%) to complete (100%) or virtually complete (≥ 80%, ≥ 90%, ≥ 95%, ≥ 96%, ≥ 97%, ≥ 98%, ≥ 99%, ≥ 99.3%, ≥ 99.7%) removal of the residual components of the medium employed or of the residual input materials resp., which have not been consumed in the fermentative process.

An abundance of technical instructions for measures a), b), c) and d) are available in the art.

Removal of water (measure a)) can be achieved inter alia by evaporation, using e.g. a falling film evaporator, by reverse osmosis or nanofiltration. The concentrates thus obtained can be further worked up by spray drying or spray granulation. It is likewise possible to dry the fermentation broth directly using spray drying or spray granulation.

Removal of the biomass (measure b)) can be achieved inter alia by centrifugation, filtration or decantation or a combination thereof.

Removal of the organic by-products (measure c)) or removal of residual components of the medium (measure d) can be achieved inter alia by chromatography, e.g. ion exchange chromatography, treatment with activated carbon or crystallization. In case the organic by-products or residual components of the medium are present in the fermentation broth as solids they can be removed by measure b).

General instructions on separation, purification and granulation methods can be found inter alia in the book of R. Ghosh "Principles of Bioseperation Engineering" (World Scientific Publishing, 2006), the book of F. J. Dechow "Seperation and Purification Techniques in Biotechnology" (Noyes Publications, 1989), the article "Bioseparation" of Shaeiwitz et al. (Ullmann's Encyclopedia of Industrial Chemistry, Wiley-VCH, 2012) and the book of P. Serno et al. "Granulieren" (Editio Cantor Verlag, 2007).

A downstream processing scheme for L-lysine products can be found in the article "L-lysine Production" of R. Kelle et al. (L. Eggeling and M. Bott (Handbook of Corynebacterium glutamicum, CRC Press, 2005)). US5279744 teaches the manufacturing of a purified L-lysine product by ion exchange chromatography. US5431933 teaches the manufacturing of dry L-amino acid products, e. g. an L-lysine product, containing most of the constituents of the fermentation broth.

Thus a concentration or purification of the L-lysine is achieved and a product having the desired content of said L-lysine is provided.

Finally the the L-lysine may be isolated from the culture broth and crystallized in form of a salt, preferably in form of the hydrochloric acid salt of L-lysine.

Analysis of L-lysine to determine its concentration at one or more time(s) during the fermentation can take place by separating the L-lysine by means of ion exchange chromatography, preferably cation exchange chromatography, with subsequent post-column derivatization using ninhydrin, as described in Spackman et al. (Analytical Chemistry 30: 1190-1206 (1958)). It is also possible to employ ortho-phthalaldehyde rather than ninhydrin for post-column derivatization. An overview article on ion exchange chromatography can be found in Pickering (LC.GC (Magazine of Chromatographic Science 7(6):484-487 (1989)). It is likewise possible to carry out a pre-column derivatization, for example using ortho-phthalaldehyde or phenyl isothiocyanate, and to fractionate the resulting amino acid derivates by reversed-phase chromatography (RP), preferably in the form of high-performance liquid chromatography (HPLC). A method of this type is described, for example, in Lindroth et al. (Analytical Chemistry 51:1167-1174 (1979)). Detection is carried out photometrically (absorption, fluorescence). A review regarding amino acid analysis can be found inter alia in the textbook "Bioanalytik" by Lottspeich and Zorbas (Spektrum Akademischer Verlag, Heidelberg, Germany 1998).

## Claims

1. A bacterium of the species *Corynebacterium glutamicum* having the ability to excrete L-lysine containing in its chromosome a polynucleotide encoding a polypeptide having the activity of a malate:quinone oxidoreductase and comprising the amino acid sequence of SEQ ID NO:2, wherein the amino acid valine at position 228 is substituted by a different proteinogenic amino acid.

2. The bacterium as claimed in claim 1, wherein said amino acid at position 228 of the amino acid sequence of SEQ ID NO:2 is aspartic acid or glutamic acid.

3. The bacterium as claimed in claims 1 or 2, wherein said amino acid at position 228 of the amino acid sequence of SEQ ID NO:2 is aspartic acid.

4. The bacterium as claimed in claim 3, wherein the polynucleotide encoding said amino acid sequence comprises the nucleotide sequence of positions 1001 to 2500 of SEQ ID NO:1 the nucleobase at position 1683 being adenine.

5. The bacterium as claimed in claim 3, wherein the polynucleotide encoding said amino acid sequence comprises the nucleotide sequence of positions 1001 to 2500 of SEQ ID NO:3.

6. The bacterium of any of the claims 1 to 5, wherein the 5'-end of the polynucleotide encoding said amino acid sequence is directly connected to the 3'-end of the polynucleotide comprising the nucleotide sequence from positions 781 to 1000 of SEQ ID NO:1 or of SEQ ID NO:3.

7. The bacterium as claimed in claim 6, wherein the polynucleotide encoding said amino acid sequence comprises the nucleotide sequence from positions 781 to 2500 of SEQ ID NO:3.

8. The bacterium as claimed in claim 6, wherein the polynucleotide encoding said amino acid sequence comprises the nucleotide sequence from positions 781 to 2500 of SEQ ID NO:1 the nucleobase at position 1683 being adenine.

9. A method for the fermentative production of an L-lysine comprising the steps of
a) cultivating the bacterium as defined in any of claims 1 to 8 in a
suitable medium under suitable conditions, and
b) accumulating L-lysine in the medium to
form an L-lysine containing fermentation broth.

10. The method of claim 9, wherein said method is selected from the group consisting of batch process, fed batch process and continuous process.

11. The method as claimed in any of the claims 9 or 10, wherein said method comprises manufacturing an L-lysine containing product from said fermentation broth.

12. The method as claimed in any of the claims 9 to 11, wherein said method comprises extracting or substantially eliminating water from said fermentation broth.

13. The method of claim 12, wherein at least 40 % (w/w), preferred at least 90 % (w/w), more preferred at least 95 % (w/w) water are extracted from the fermentation broth.

14. The method of claim 11, wherein said manufacturing comprises a purification step.

15. The method of claim 14, wherein said purification step is selected from the group consisting of treatment with char coal, ionic exchange and crystallization.

16. The method of claim 9, further comprising isolation of L-lysine from the fermentation broth and crystallization of L-lysine in form of a salt.


SEQUENCE LISTING

<110>  Evonik Degussa GmbH

<120>  Method for the fermentative production of L-lysine

<130>  filing number: 201700371

<160>  8

<170>  PatentIn version 3.5

<210>  1
<211>  3503
<212>  DNA
<213>  Corynebacterium glutamicum ATCC13032



<220>
<221>  misc_feature
<222>  (1)..(3503)
<223>  nucleotide sequence comprising locus_tag NCgl1926 disclosed in
       GenBank accession number NC_003450

<220>
<221>  misc_feature
<222>  (781)..(781)
<223>  nucleobase adenine

<220>
<221>  CDS
<222>  (1001)..(2500)

<220>
<221>  misc_feature
<222>  (1682)..(1684)
<223>  gtc codon for valine at position 228

<220>
<221>  misc_feature
<222>  (1683)..(1683)
<223>  nucleobase thymine

<220>
<221>  misc_feature
<222>  (2228)..(2230)
<223>  ttc codon for phenylalanine at position 410

<220>
<221>  misc_feature
<222>  (2230)..(2230)
<223>  nucleobase cytosine

<220>
<221>  misc_feature
<222>  (2501)..(2503)
<223>  stop codon

<400>  1
tccgtatgct cccaaacctc cgcctgggat gatggttgtg ggggagcgtt tgcccaacac      60

cctcaccata ggggtgatca gcttgatgaa cagtggtgga tacatcatgt ccagccacgg     120

actgttgagg accagcgctg gaatcttctc aatggcagct ggattgcttg tgcgcatctg 180

ggacatccac agaggaacga tgagtccacc agtggaatgg gcgacgggga ctagctcagg 240

gtgggtggaa gagatgacct cggcagcagc tgttaagtca gggaagtaat gggcaagatc 300

agaggtgtag tgccactgct gtcctggacg gtaggagcgt ccacattttc taagatcaat 360

gccgtacaca gcaaaaccgg cattgtggaa aaactccgcg aattcagtgt ggaagaagta 420

gtccgtcatg ccgtgaaccc acagcaacgc tgggcgggca gcaaaagact cgtccgcgtg 480

attgtctggg ttgtagcgca caaccgttgt cacaacatct gtttcattat ccgggtcgtc 540

gccgagctcg atggtgaggt tttggtagcc ctctcccagg aagtctggtt tccactgctg 600

catctgattc atacgtataa cgataggtcg attgttggtg tggtgtgcgc gagtcgactg 660

aaatgttcac gtggtgaaac ttccgcgata ctactcatgt ttgcgaattg cacatttact 720

aactttgcaa attgggggag ggggtagcgc gggggaggaa ttcgcatgag aaaggggaat 780

atcccgtgct tgtttattca gctcgaggtg gcaggcgtac actctatatt cacggacaat 840

gtgtacccac gctttcttgt aagaaacaag aagggtaacg ccccacgcgt cagtcaaaaa 900

tatggccaac acttgcattc gggtgctggc gatcatttat gagatgacgc cttgtgttgg 960

tgttcggcag agaactcgcg gagataaaag gaagttgaac atg tca gat tcc ccg 1015
Met Ser Asp Ser Pro
1               5

aag aac gca ccg agg att acc gat gag gca gat gta gtt ctc att ggt 1063
Lys Asn Ala Pro Arg Ile Thr Asp Glu Ala Asp Val Val Leu Ile Gly
                10                  15                  20

gcc ggt atc atg agc tcc acg ctg ggt gca atg ctg cgt cag ctg gag 1111
Ala Gly Ile Met Ser Ser Thr Leu Gly Ala Met Leu Arg Gln Leu Glu
            25                  30                  35

cca agc tgg act cag atc gtc ttc gag cgt ttg gat gga ccg gca caa 1159
Pro Ser Trp Thr Gln Ile Val Phe Glu Arg Leu Asp Gly Pro Ala Gln
        40                  45                  50

gag tcg tcc tcc ccg tgg aac aat gca gga acc ggc cac tct gct cta 1207
Glu Ser Ser Ser Pro Trp Asn Asn Ala Gly Thr Gly His Ser Ala Leu
    55                  60                  65

tgc gag ctg aac tac acc cca gag gtt aag ggc aag gtt gaa att gcc 1255
Cys Glu Leu Asn Tyr Thr Pro Glu Val Lys Gly Lys Val Glu Ile Ala
70                  75                  80                  85

aag gct gta gga atc aac gag aag ttc cag gtt tcc cgt cag ttc tgg 1303
Lys Ala Val Gly Ile Asn Glu Lys Phe Gln Val Ser Arg Gln Phe Trp
                90                  95                  100

tct cac ctc gtt gaa gag gga gtg ctg tct gat cct aag gaa ttc atc 1351
Ser His Leu Val Glu Glu Gly Val Leu Ser Asp Pro Lys Glu Phe Ile
            105                 110                 115

aac cct gtt cct cac gta tct ttc ggc cag ggc gca gat cag gtt gca 1399
Asn Pro Val Pro His Val Ser Phe Gly Gln Gly Ala Asp Gln Val Ala
        120                 125                 130

tac atc aag gct cgc tac gaa gct ttg aag gat cac cca ctc ttc cag 1447
Tyr Ile Lys Ala Arg Tyr Glu Ala Leu Lys Asp His Pro Leu Phe Gln
    135                 140                 145

ggc atg acc tac gct gac gat gaa gct acc ttc acc gag aag ctg cct 1495
Gly Met Thr Tyr Ala Asp Asp Glu Ala Thr Phe Thr Glu Lys Leu Pro
150                 155                 160                 165

ttg atg gca aag ggc cgt gac ttc tct gat cca gta gca atc tct tgg 1543
Leu Met Ala Lys Gly Arg Asp Phe Ser Asp Pro Val Ala Ile Ser Trp
                170                 175                 180

atc gat gaa ggc acc gac atc aac tac ggt gct cag acc aag cag tac 1591
Ile Asp Glu Gly Thr Asp Ile Asn Tyr Gly Ala Gln Thr Lys Gln Tyr
            185                 190                 195

ctg gat gca gct gaa gtt gaa ggc act gaa atc cgc tat ggc cac gaa 1639
Leu Asp Ala Ala Glu Val Glu Gly Thr Glu Ile Arg Tyr Gly His Glu
        200                 205                 210

gtc aag agc atc aag gct gat ggc gca aag tgg atc gtg acc gtc aag 1687
Val Lys Ser Ile Lys Ala Asp Gly Ala Lys Trp Ile Val Thr Val Lys
    215                 220                 225

aac gta cac act ggc gac acc aag acc atc aag gca aac ttc gtg ttc 1735
Asn Val His Thr Gly Asp Thr Lys Thr Ile Lys Ala Asn Phe Val Phe
230                 235                 240                 245

gtc ggc gca ggc gga tac gca ctg gat ctg ctt cgc agc gca ggc atc 1783
Val Gly Ala Gly Gly Tyr Ala Leu Asp Leu Leu Arg Ser Ala Gly Ile
                250                 255                 260

cca cag gtc aag ggc ttc gct gga ttc cca gta tcc ggc ctg tgg ctt 1831
Pro Gln Val Lys Gly Phe Ala Gly Phe Pro Val Ser Gly Leu Trp Leu
            265                 270                 275

cgt tgc acc aac gag gaa ctg atc gag cag cac gca gcc aag gta tat 1879
Arg Cys Thr Asn Glu Glu Leu Ile Glu Gln His Ala Ala Lys Val Tyr
        280                 285                 290

ggc aag gca tct gtt ggc gct cct cca atg tct gtt cct cac ctt gac 1927
Gly Lys Ala Ser Val Gly Ala Pro Pro Met Ser Val Pro His Leu Asp
    295                 300                 305

acc cgc gtt atc gag ggt gaa aag ggt ctg ctc ttt gga cct tac ggt 1975
Thr Arg Val Ile Glu Gly Glu Lys Gly Leu Leu Phe Gly Pro Tyr Gly
310                 315                 320                 325

ggc tgg acc cct aag ttc ttg aag gaa ggc tcc tac ctg gac ctg ttc 2023
Gly Trp Thr Pro Lys Phe Leu Lys Glu Gly Ser Tyr Leu Asp Leu Phe
                330                 335                 340

aag tcc atc cgc cca gac aac att cct tcc tac ctt ggc gtt gct gct 2071
Lys Ser Ile Arg Pro Asp Asn Ile Pro Ser Tyr Leu Gly Val Ala Ala
            345                 350                 355

cag gaa ttt gat ctg acc aag tac ctt gtc act gaa gtt ctc aag gac 2119
Gln Glu Phe Asp Leu Thr Lys Tyr Leu Val Thr Glu Val Leu Lys Asp
        360                 365                 370

cag gac aag cgt atg gat gct ctt cgc gag tac atg cca gag gca caa 2167
Gln Asp Lys Arg Met Asp Ala Leu Arg Glu Tyr Met Pro Glu Ala Gln

375                 380                 385

aac ggc gat tgg gag acc atc gtt gcc gga cag cgt gtt cag gtt att        2215
Asn Gly Asp Trp Glu Thr Ile Val Ala Gly Gln Arg Val Gln Val Ile
390                 395                 400                 405

aag cct gca gga ttc cct aag ttc ggt tcc ctg gaa ttc ggc acc acc        2263
Lys Pro Ala Gly Phe Pro Lys Phe Gly Ser Leu Glu Phe Gly Thr Thr
                410                 415                 420

ttg atc aac aac tcc gaa ggc acc atc gcc gga ttg ctc ggt gct tcc        2311
Leu Ile Asn Asn Ser Glu Gly Thr Ile Ala Gly Leu Leu Gly Ala Ser
            425                 430                 435

cct gga gca tcc atc gca cct tcc gca atg atc gag ctg ctt gag cgt        2359
Pro Gly Ala Ser Ile Ala Pro Ser Ala Met Ile Glu Leu Leu Glu Arg
        440                 445                 450

tgc ttc ggt gac cgc atg atc gag tgg ggc gac aag ctg aag gac atg        2407
Cys Phe Gly Asp Arg Met Ile Glu Trp Gly Asp Lys Leu Lys Asp Met
    455                 460                 465

atc cct tcc tac ggc aag aag ctt gct tcc gag cca gca ctg ttt gag        2455
Ile Pro Ser Tyr Gly Lys Lys Leu Ala Ser Glu Pro Ala Leu Phe Glu
470                 475                 480                 485

cag cag tgg gca cgc acc cag aag acc ctg aag ctt gag gaa gcc            2500
Gln Gln Trp Ala Arg Thr Gln Lys Thr Leu Lys Leu Glu Glu Ala
                490                 495                 500

taaatcttct aactgctttc tttaaagcac ccgcacatgt ctgttgaggt ttcacctgcg      2560

gagacaatct ccgccttcat gggttggaac tgacacagtt gaaggcatgt cgggtgcttt      2620

gcgtattctt tgccagtgtg atttaggcga caccaataat ttattgggta tccaccaatt      2680

accgctgtga gcactgcaaa ttacgtattc gaaaagccat gtccaccacg tgttctatcc      2740

tggcggcatg caaaaaatca ccccaaacat ctggtgccaa ggcaccgcag acgaagcagc      2800

cgaattctac gtcaatgcgt tttctgagtt tccgggtggc gcagaagtac tcaccacagt      2860

taagtatccc gaagctggct tgctggactt ccaggagcct ttcgcaggaa aaaccttgac      2920

ggtggaactc gctatctcag gctttaagat catcttgatc aatgctggtg aagagttcac      2980

tcccaaccca tcgatcagct tcatggtgaa ttttgatgcg gtgcgtgatg aaaatgccaa      3040

agagcacctt gatgcggtgt gggaaaaact ccatgaaggc ggcagcacac tgatgccagt      3100

cgatacttac ccattttcgg aatactacgg gtgggtacaa gacaaatatg gtgtgagctg      3160

gcaattgatg ctcagccgcc cagaagaaaa gccaggtccc gcagtaatcc caacgctctt      3220

atttggtggg gcagctcaaa atcaggcagg cccagctcaa gaaaactacg ttgaggtgtt      3280

cccgaactcc caacttggtg atcgtgcacc ttatggacag caaacaggtc ctgccactcc      3340

tgaggccctc atgttttccc agttccaact cgacggtcag tggattttcg cgatggattc      3400

cggagttgag caagatttca ccttcagtga gggtgtctca ttgatgtatg aagctcatgg      3460

tcaagaagaa ctcgatgcca tctggaatgc actctcggca gtt                        3503

<210>  2
<211>  500
<212>  PRT
<213>  Corynebacterium glutamicum ATCC13032

<400>  2

Met Ser Asp Ser Pro Lys Asn Ala Pro Arg Ile Thr Asp Glu Ala Asp
1               5                   10                  15

Val Val Leu Ile Gly Ala Gly Ile Met Ser Ser Thr Leu Gly Ala Met
            20                  25                  30

Leu Arg Gln Leu Glu Pro Ser Trp Thr Gln Ile Val Phe Glu Arg Leu
        35                  40                  45

Asp Gly Pro Ala Gln Glu Ser Ser Ser Pro Trp Asn Asn Ala Gly Thr
    50                  55                  60

Gly His Ser Ala Leu Cys Glu Leu Asn Tyr Thr Pro Glu Val Lys Gly
65                  70                  75                  80

Lys Val Glu Ile Ala Lys Ala Val Gly Ile Asn Glu Lys Phe Gln Val
                85                  90                  95

Ser Arg Gln Phe Trp Ser His Leu Val Glu Glu Gly Val Leu Ser Asp
            100                 105                 110

Pro Lys Glu Phe Ile Asn Pro Val Pro His Val Ser Phe Gly Gln Gly
        115                 120                 125

Ala Asp Gln Val Ala Tyr Ile Lys Ala Arg Tyr Glu Ala Leu Lys Asp
    130                 135                 140

His Pro Leu Phe Gln Gly Met Thr Tyr Ala Asp Asp Glu Ala Thr Phe
145                 150                 155                 160

Thr Glu Lys Leu Pro Leu Met Ala Lys Gly Arg Asp Phe Ser Asp Pro
                165                 170                 175

Val Ala Ile Ser Trp Ile Asp Glu Gly Thr Asp Ile Asn Tyr Gly Ala
            180                 185                 190

Gln Thr Lys Gln Tyr Leu Asp Ala Ala Glu Val Glu Gly Thr Glu Ile
        195                 200                 205

Arg Tyr Gly His Glu Val Lys Ser Ile Lys Ala Asp Gly Ala Lys Trp
    210                 215                 220

Ile Val Thr Val Lys Asn Val His Thr Gly Asp Thr Lys Thr Ile Lys
225                 230                 235                 240

Ala Asn Phe Val Phe Val Gly Ala Gly Gly Tyr Ala Leu Asp Leu Leu
                245                 250                 255

Arg Ser Ala Gly Ile Pro Gln Val Lys Gly Phe Ala Gly Phe Pro Val
            260                 265                 270

Ser Gly Leu Trp Leu Arg Cys Thr Asn Glu Glu Leu Ile Glu Gln His
        275                 280                 285

Ala Ala Lys Val Tyr Gly Lys Ala Ser Val Gly Ala Pro Pro Met Ser
    290                 295                 300

Val Pro His Leu Asp Thr Arg Val Ile Glu Gly Glu Lys Gly Leu Leu
305                 310                 315                 320

Phe Gly Pro Tyr Gly Gly Trp Thr Pro Lys Phe Leu Lys Glu Gly Ser
                325                 330                 335

Tyr Leu Asp Leu Phe Lys Ser Ile Arg Pro Asp Asn Ile Pro Ser Tyr
            340                 345                 350

Leu Gly Val Ala Ala Gln Glu Phe Asp Leu Thr Lys Tyr Leu Val Thr
        355                 360                 365

Glu Val Leu Lys Asp Gln Asp Lys Arg Met Asp Ala Leu Arg Glu Tyr
    370                 375                 380

Met Pro Glu Ala Gln Asn Gly Asp Trp Glu Thr Ile Val Ala Gly Gln
385                 390                 395                 400

Arg Val Gln Val Ile Lys Pro Ala Gly Phe Pro Lys Phe Gly Ser Leu
                405                 410                 415

Glu Phe Gly Thr Thr Leu Ile Asn Asn Ser Glu Gly Thr Ile Ala Gly
            420                 425                 430

Leu Leu Gly Ala Ser Pro Gly Ala Ser Ile Ala Pro Ser Ala Met Ile
        435                 440                 445

Glu Leu Leu Glu Arg Cys Phe Gly Asp Arg Met Ile Glu Trp Gly Asp
    450                 455                 460

Lys Leu Lys Asp Met Ile Pro Ser Tyr Gly Lys Lys Leu Ala Ser Glu

465                 470                 475                 480

Pro Ala Leu Phe Glu Gln Gln Trp Ala Arg Thr Gln Lys Thr Leu Lys
                485                 490                 495

Leu Glu Glu Ala
            500

<210>  3
<211>  3503
<212>  DNA
<213>  Corynebacterium glutamicum

<220>
<221>  misc_feature
<222>  (781)..(781)
<223>  nucleobase adenine

<220>
<221>  CDS
<222>  (1001)..(2500)

<220>
<221>  misc_feature
<222>  (1682)..(1684)
<223>  gac codon for aspartic acid at position 228

<220>
<221>  mutation
<222>  (1683)..(1683)
<223>  nucleobase adenine

<220>
<221>  misc_feature
<222>  (2228)..(2230)
<223>  ttt codon for phenylalanine at position 410

<220>
<221>  misc_feature
<222>  (2230)..(2230)
<223>  nucleobase thymine

<220>
<221>  misc_feature
<222>  (2501)..(2503)
<223>  stop codon

<400>  3
tccgtatgct cccaaacctc cgcctgggat gatggttgtg ggggagcgtt tgcccaacac      60

cctcaccata ggggtgatca gcttgatgaa cagtggtgga tacatcatgt ccagccacgg     120

actgttgagg accagcgctg gaatcttctc aatggcagct ggattgcttg tgcgcatctg     180

ggacatccac agaggaacga tgagtccacc agtggaatgg gcgacgggga ctagctcagg     240

gtgggtggaa gagatgacct cggcagcagc tgttaagtca gggaagtaat gggcaagatc     300

agaggtgtag tgccactgct gtcctggacg gtaggagcgt ccacattttc taagatcaat     360

gccgtacaca gcaaaaccgg cattgtggaa aaactccgcg aattcagtgt ggaagaagta 420

gtccgtcatg ccgtgaaccc acagcaacgc tgggcgggca gcaaaagact cgtccgcgtg 480

attgtctggg ttgtagcgca caaccgttgt cacaacatct gtttcattat ccgggtcgtc 540

gccgagctcg atggtgaggt tttggtagcc ctctcccagg aagtctggtt tccactgctg 600

catctgattc atacgtataa cgataggtcg attgttggtg tggtgtgcgc gagtcgactg 660

aaatgttcac gtggtgaaac ttccgcgata ctactcatgt ttgcgaattg cacatttact 720

aactttgcaa attgggggag ggggtagcgc gggggaggaa ttcgcatgag aaaggggaat 780

atcccgtgct tgtttattca gctcgaggtg gcaggcgtac actctatatt cacggacaat 840

gtgtacccac gctttcttgt aagaaacaag aagggtaacg ccccacgcgt cagtcaaaaa 900

tatggccaac acttgcattc gggtgctggc gatcatttat gagatgacgc cttgtgttgg 960

tgttcggcag agaactcgcg gagataaaag gaagttgaac atg tca gat tcc ccg 1015
Met Ser Asp Ser Pro
1               5

aag aac gca ccg agg att acc gat gag gca gat gta gtt ctc att ggt 1063
Lys Asn Ala Pro Arg Ile Thr Asp Glu Ala Asp Val Val Leu Ile Gly
                10                  15                  20

gcc ggt atc atg agc tcc acg ctg ggt gca atg ctg cgt cag ctg gag 1111
Ala Gly Ile Met Ser Ser Thr Leu Gly Ala Met Leu Arg Gln Leu Glu
            25                  30                  35

cca agc tgg act cag atc gtc ttc gag cgt ttg gat gga ccg gca caa 1159
Pro Ser Trp Thr Gln Ile Val Phe Glu Arg Leu Asp Gly Pro Ala Gln
        40                  45                  50

gag tcg tcc tcc ccg tgg aac aat gca gga acc ggc cac tct gct cta 1207
Glu Ser Ser Ser Pro Trp Asn Asn Ala Gly Thr Gly His Ser Ala Leu
    55                  60                  65

tgc gag ctg aac tac acc cca gag gtt aag ggc aag gtt gaa att gcc 1255
Cys Glu Leu Asn Tyr Thr Pro Glu Val Lys Gly Lys Val Glu Ile Ala
70                  75                  80                  85

aag gct gta gga atc aac gag aag ttc cag gtt tcc cgt cag ttc tgg 1303
Lys Ala Val Gly Ile Asn Glu Lys Phe Gln Val Ser Arg Gln Phe Trp
                90                  95                  100

tct cac ctc gtt gaa gag gga gtg ctg tct gat cct aag gaa ttc atc 1351
Ser His Leu Val Glu Glu Gly Val Leu Ser Asp Pro Lys Glu Phe Ile
            105                 110                 115

aac cct gtt cct cac gta tct ttc ggc cag ggc gca gat cag gtt gca 1399
Asn Pro Val Pro His Val Ser Phe Gly Gln Gly Ala Asp Gln Val Ala
        120                 125                 130

tac atc aag gct cgc tac gaa gct ttg aag gat cac cca ctc ttc cag 1447
Tyr Ile Lys Ala Arg Tyr Glu Ala Leu Lys Asp His Pro Leu Phe Gln
    135                 140                 145

ggc atg acc tac gct gac gat gaa gct acc ttc acc gag aag ctg cct 1495
Gly Met Thr Tyr Ala Asp Asp Glu Ala Thr Phe Thr Glu Lys Leu Pro

150                 155                 160                 165

ttg atg gca aag ggc cgt gac ttc tct gat cca gta gca atc tct tgg      1543
Leu Met Ala Lys Gly Arg Asp Phe Ser Asp Pro Val Ala Ile Ser Trp
                170                 175                 180

atc gat gaa ggc acc gac atc aac tac ggt gct cag acc aag cag tac      1591
Ile Asp Glu Gly Thr Asp Ile Asn Tyr Gly Ala Gln Thr Lys Gln Tyr
            185                 190                 195

ctg gat gca gct gaa gtt gaa ggc act gaa atc cgc tat ggc cac gaa      1639
Leu Asp Ala Ala Glu Val Glu Gly Thr Glu Ile Arg Tyr Gly His Glu
        200                 205                 210

gtc aag agc atc aag gct gat ggc gca aag tgg atc gtg acc gac aag      1687
Val Lys Ser Ile Lys Ala Asp Gly Ala Lys Trp Ile Val Thr Asp Lys
    215                 220                 225

aac gta cac act ggc gac acc aag acc atc aag gca aac ttc gtg ttc      1735
Asn Val His Thr Gly Asp Thr Lys Thr Ile Lys Ala Asn Phe Val Phe
230                 235                 240                 245

gtc ggc gca ggc gga tac gca ctg gat ctg ctt cgc agc gca ggc atc      1783
Val Gly Ala Gly Gly Tyr Ala Leu Asp Leu Leu Arg Ser Ala Gly Ile
                250                 255                 260

cca cag gtc aag ggc ttc gct gga ttc cca gta tcc ggc ctg tgg ctt      1831
Pro Gln Val Lys Gly Phe Ala Gly Phe Pro Val Ser Gly Leu Trp Leu
            265                 270                 275

cgt tgc acc aac gag gaa ctg atc gag cag cac gca gcc aag gta tat      1879
Arg Cys Thr Asn Glu Glu Leu Ile Glu Gln His Ala Ala Lys Val Tyr
        280                 285                 290

ggc aag gca tct gtt ggc gct cct cca atg tct gtt cct cac ctt gac      1927
Gly Lys Ala Ser Val Gly Ala Pro Pro Met Ser Val Pro His Leu Asp
    295                 300                 305

acc cgc gtt atc gag ggt gaa aag ggt ctg ctc ttt gga cct tac ggt      1975
Thr Arg Val Ile Glu Gly Glu Lys Gly Leu Leu Phe Gly Pro Tyr Gly
310                 315                 320                 325

ggc tgg acc cct aag ttc ttg aag gaa ggc tcc tac ctg gac ctg ttc      2023
Gly Trp Thr Pro Lys Phe Leu Lys Glu Gly Ser Tyr Leu Asp Leu Phe
                330                 335                 340

aag tcc atc cgc cca gac aac att cct tcc tac ctt ggc gtt gct gct      2071
Lys Ser Ile Arg Pro Asp Asn Ile Pro Ser Tyr Leu Gly Val Ala Ala
            345                 350                 355

cag gaa ttt gat ctg acc aag tac ctt gtc act gaa gtt ctc aag gac      2119
Gln Glu Phe Asp Leu Thr Lys Tyr Leu Val Thr Glu Val Leu Lys Asp
        360                 365                 370

cag gac aag cgt atg gat gct ctt cgc gag tac atg cca gag gca caa      2167
Gln Asp Lys Arg Met Asp Ala Leu Arg Glu Tyr Met Pro Glu Ala Gln
    375                 380                 385

aac ggc gat tgg gag acc atc gtt gcc gga cag cgt gtt cag gtt att      2215
Asn Gly Asp Trp Glu Thr Ile Val Ala Gly Gln Arg Val Gln Val Ile
390                 395                 400                 405

aag cct gca gga ttt cct aag ttc ggt tcc ctg gaa ttc ggc acc acc      2263

Lys Pro Ala Gly Phe Pro Lys Phe Gly Ser Leu Glu Phe Gly Thr Thr
                410                 415                 420

ttg atc aac aac tcc gaa ggc acc atc gcc gga ttg ctc ggt gct tcc      2311
Leu Ile Asn Asn Ser Glu Gly Thr Ile Ala Gly Leu Leu Gly Ala Ser
            425                 430                 435

cct gga gca tcc atc gca cct tcc gca atg atc gag ctg ctt gag cgt      2359
Pro Gly Ala Ser Ile Ala Pro Ser Ala Met Ile Glu Leu Leu Glu Arg
        440                 445                 450

tgc ttc ggt gac cgc atg atc gag tgg ggc gac aag ctg aag gac atg      2407
Cys Phe Gly Asp Arg Met Ile Glu Trp Gly Asp Lys Leu Lys Asp Met
    455                 460                 465

atc cct tcc tac ggc aag aag ctt gct tcc gag cca gca ctg ttt gag      2455
Ile Pro Ser Tyr Gly Lys Lys Leu Ala Ser Glu Pro Ala Leu Phe Glu
470                 475                 480                 485

cag cag tgg gca cgc acc cag aag acc ctg aag ctt gag gaa gcc          2500
Gln Gln Trp Ala Arg Thr Gln Lys Thr Leu Lys Leu Glu Glu Ala
                490                 495                 500

taaatcttct aactgctttc tttaaagcac ccgcacatgt ctgttgaggt ttcacctgcg      2560

gagacaatct ccgccttcat gggttggaac tgacacagtt gaaggcatgt cgggtgcttt      2620

gcgtattctt tgccagtgtg atttaggcga caccaataat ttattgggta tccaccaatt      2680

accgctgtga gcactgcaaa ttacgtattc gaaaagccat gtccaccacg tgttctatcc      2740

tggcggcatg caaaaaatca ccccaaacat ctggtgccaa ggcaccgcag acgaagcagc      2800

cgaattctac gtcaatgcgt tttctgagtt tccgggtggc gcagaagtac tcaccacagt      2860

taagtatccc gaagctggct tgctggactt ccaggagcct ttcgcaggaa aaaccttgac      2920

ggtggaactc gctatctcag gctttaagat catcttgatc aatgctggtg aagagttcac      2980

tcccaaccca tcgatcagct tcatggtgaa ttttgatgcg gtgcgtgatg aaaatgccaa      3040

agagcacctt gatgcggtgt gggaaaaact ccatgaaggc ggcagcacac tgatgccagt      3100

cgatacttac ccattttcgg aatactacgg gtgggtacaa gacaaatatg gtgtgagctg      3160

gcaattgatg ctcagccgcc cagaagaaaa gccaggtccc gcagtaatcc caacgctctt      3220

atttggtggg gcagctcaaa atcaggcagg cccagctcaa gaaaactacg ttgaggtgtt      3280

cccgaactcc caacttggtg atcgtgcacc ttatggacag caaacaggtc ctgccactcc      3340

tgaggccctc atgttttccc agttccaact cgacggtcag tggattttcg cgatggattc      3400

cggagttgag caagatttca ccttcagtga gggtgtctca ttgatgtatg aagctcatgg      3460

tcaagaagaa ctcgatgcca tctggaatgc actctcggca gtt                        3503

<210>  4
<211>  500
<212>  PRT
<213>  Corynebacterium glutamicum

<400> 4

Met Ser Asp Ser Pro Lys Asn Ala Pro Arg Ile Thr Asp Glu Ala Asp
1               5                   10                  15

Val Val Leu Ile Gly Ala Gly Ile Met Ser Ser Thr Leu Gly Ala Met
            20                  25                  30

Leu Arg Gln Leu Glu Pro Ser Trp Thr Gln Ile Val Phe Glu Arg Leu
        35                  40                  45

Asp Gly Pro Ala Gln Glu Ser Ser Ser Pro Trp Asn Asn Ala Gly Thr
    50                  55                  60

Gly His Ser Ala Leu Cys Glu Leu Asn Tyr Thr Pro Glu Val Lys Gly
65                  70                  75                  80

Lys Val Glu Ile Ala Lys Ala Val Gly Ile Asn Glu Lys Phe Gln Val
                85                  90                  95

Ser Arg Gln Phe Trp Ser His Leu Val Glu Glu Gly Val Leu Ser Asp
            100                 105                 110

Pro Lys Glu Phe Ile Asn Pro Val Pro His Val Ser Phe Gly Gln Gly
        115                 120                 125

Ala Asp Gln Val Ala Tyr Ile Lys Ala Arg Tyr Glu Ala Leu Lys Asp
    130                 135                 140

His Pro Leu Phe Gln Gly Met Thr Tyr Ala Asp Asp Glu Ala Thr Phe
145                 150                 155                 160

Thr Glu Lys Leu Pro Leu Met Ala Lys Gly Arg Asp Phe Ser Asp Pro
                165                 170                 175

Val Ala Ile Ser Trp Ile Asp Glu Gly Thr Asp Ile Asn Tyr Gly Ala
            180                 185                 190

Gln Thr Lys Gln Tyr Leu Asp Ala Ala Glu Val Glu Gly Thr Glu Ile
        195                 200                 205

Arg Tyr Gly His Glu Val Lys Ser Ile Lys Ala Asp Gly Ala Lys Trp
    210                 215                 220

Ile Val Thr Asp Lys Asn Val His Thr Gly Asp Thr Lys Thr Ile Lys
225                 230                 235                 240

Ala Asn Phe Val Phe Val Gly Ala Gly Gly Tyr Ala Leu Asp Leu Leu

245                 250                 255

Arg Ser Ala Gly Ile Pro Gln Val Lys Gly Phe Ala Gly Phe Pro Val
            260                 265                 270

Ser Gly Leu Trp Leu Arg Cys Thr Asn Glu Glu Leu Ile Glu Gln His
        275                 280                 285

Ala Ala Lys Val Tyr Gly Lys Ala Ser Val Gly Ala Pro Pro Met Ser
    290                 295                 300

Val Pro His Leu Asp Thr Arg Val Ile Glu Gly Glu Lys Gly Leu Leu
305                 310                 315                 320

Phe Gly Pro Tyr Gly Gly Trp Thr Pro Lys Phe Leu Lys Glu Gly Ser
                325                 330                 335

Tyr Leu Asp Leu Phe Lys Ser Ile Arg Pro Asp Asn Ile Pro Ser Tyr
            340                 345                 350

Leu Gly Val Ala Ala Gln Glu Phe Asp Leu Thr Lys Tyr Leu Val Thr
        355                 360                 365

Glu Val Leu Lys Asp Gln Asp Lys Arg Met Asp Ala Leu Arg Glu Tyr
    370                 375                 380

Met Pro Glu Ala Gln Asn Gly Asp Trp Glu Thr Ile Val Ala Gly Gln
385                 390                 395                 400

Arg Val Gln Val Ile Lys Pro Ala Gly Phe Pro Lys Phe Gly Ser Leu
                405                 410                 415

Glu Phe Gly Thr Thr Leu Ile Asn Asn Ser Glu Gly Thr Ile Ala Gly
            420                 425                 430

Leu Leu Gly Ala Ser Pro Gly Ala Ser Ile Ala Pro Ser Ala Met Ile
        435                 440                 445

Glu Leu Leu Glu Arg Cys Phe Gly Asp Arg Met Ile Glu Trp Gly Asp
    450                 455                 460

Lys Leu Lys Asp Met Ile Pro Ser Tyr Gly Lys Lys Leu Ala Ser Glu
465                 470                 475                 480

Pro Ala Leu Phe Glu Gln Gln Trp Ala Arg Thr Gln Lys Thr Leu Lys
                485                 490                 495

Leu Glu Glu Ala
            500

<210>  5
<211>  1266
<212>  DNA
<213>  Corynebacterium glutamicum ATCC13032

<220>
<221>  CDS
<222>  (1)..(1263)

<400>  5
gtg gcc ctg gtc gta cag aaa tat ggc ggt tcc tcg ctt gag agt gcg      48
Met Ala Leu Val Val Gln Lys Tyr Gly Gly Ser Ser Leu Glu Ser Ala
1               5                   10                  15

gaa cgc att aga aac gtc gct gaa cgg atc gtt gcc acc aag aag gct      96
Glu Arg Ile Arg Asn Val Ala Glu Arg Ile Val Ala Thr Lys Lys Ala
            20                  25                  30

gga aat gat gtc gtg gtt gtc tgc tcc gca atg gga gac acc acg gat      144
Gly Asn Asp Val Val Val Val Cys Ser Ala Met Gly Asp Thr Thr Asp
        35                  40                  45

gaa ctt cta gaa ctt gca gcg gca gtg aat ccc gtt ccg cca gct cgt      192
Glu Leu Leu Glu Leu Ala Ala Ala Val Asn Pro Val Pro Pro Ala Arg
    50                  55                  60

gaa atg gat atg ctc ctg act gct ggt gag cgt att tct aac gct ctc      240
Glu Met Asp Met Leu Leu Thr Ala Gly Glu Arg Ile Ser Asn Ala Leu
65                  70                  75                  80

gtc gcc atg gct att gag tcc ctt ggc gca gaa gcc caa tct ttc acg      288
Val Ala Met Ala Ile Glu Ser Leu Gly Ala Glu Ala Gln Ser Phe Thr
                85                  90                  95

ggc tct cag gct ggt gtg ctc acc acc gag cgc cac gga aac gca cgc      336
Gly Ser Gln Ala Gly Val Leu Thr Thr Glu Arg His Gly Asn Ala Arg
            100                 105                 110

att gtt gat gtc act cca ggt cgt gtg cgt gaa gca ctc gat gag ggc      384
Ile Val Asp Val Thr Pro Gly Arg Val Arg Glu Ala Leu Asp Glu Gly
        115                 120                 125

aag atc tgc att gtt gct ggt ttc cag ggt gtt aat aaa gaa acc cgc      432
Lys Ile Cys Ile Val Ala Gly Phe Gln Gly Val Asn Lys Glu Thr Arg
    130                 135                 140

gat gtc acc acg ttg ggt cgt ggt ggt tct gac acc act gca gtt gcg      480
Asp Val Thr Thr Leu Gly Arg Gly Gly Ser Asp Thr Thr Ala Val Ala
145                 150                 155                 160

ttg gca gct gct ttg aac gct gat gtg tgt gag att tac tcg gac gtt      528
Leu Ala Ala Ala Leu Asn Ala Asp Val Cys Glu Ile Tyr Ser Asp Val
                165                 170                 175

gac ggt gtg tat acc gct gac ccg cgc atc gtt cct aat gca cag aag      576
Asp Gly Val Tyr Thr Ala Asp Pro Arg Ile Val Pro Asn Ala Gln Lys
            180                 185                 190

ctg gaa aag ctc agc ttc gaa gaa atg ctg gaa ctt gct gct gtt ggc 624
Leu Glu Lys Leu Ser Phe Glu Glu Met Leu Glu Leu Ala Ala Val Gly
        195                 200                 205

tcc aag att ttg gtg ctg cgc agt gtt gaa tac gct cgt gca ttc aat 672
Ser Lys Ile Leu Val Leu Arg Ser Val Glu Tyr Ala Arg Ala Phe Asn
    210                 215                 220

gtg cca ctt cgc gta cgc tcg tct tat agt aat gat ccc ggc act ttg 720
Val Pro Leu Arg Val Arg Ser Ser Tyr Ser Asn Asp Pro Gly Thr Leu
225                 230                 235                 240

att gcc ggc tct atg gag gat att cct gtg gaa gaa gca gtc ctt acc 768
Ile Ala Gly Ser Met Glu Asp Ile Pro Val Glu Glu Ala Val Leu Thr
                245                 250                 255

ggt gtc gca acc gac aag tcc gaa gcc aaa gta acc gtt ctg ggt att 816
Gly Val Ala Thr Asp Lys Ser Glu Ala Lys Val Thr Val Leu Gly Ile
            260                 265                 270

tcc gat aag cca ggc gag gct gcg aag gtt ttc cgt gcg ttg gct gat 864
Ser Asp Lys Pro Gly Glu Ala Ala Lys Val Phe Arg Ala Leu Ala Asp
        275                 280                 285

gca gaa atc aac att gac atg gtt ctg cag aac gtc tct tct gta gaa 912
Ala Glu Ile Asn Ile Asp Met Val Leu Gln Asn Val Ser Ser Val Glu
    290                 295                 300

gac ggc acc acc gac atc acc ttc acc tgc cct cgt tcc gac ggc cgc 960
Asp Gly Thr Thr Asp Ile Thr Phe Thr Cys Pro Arg Ser Asp Gly Arg
305                 310                 315                 320

cgc gcg atg gag atc ttg aag aag ctt cag gtt cag ggc aac tgg acc 1008
Arg Ala Met Glu Ile Leu Lys Lys Leu Gln Val Gln Gly Asn Trp Thr
                325                 330                 335

aat gtg ctt tac gac gac cag gtc ggc aaa gtc tcc ctc gtg ggt gct 1056
Asn Val Leu Tyr Asp Asp Gln Val Gly Lys Val Ser Leu Val Gly Ala
            340                 345                 350

ggc atg aag tct cac cca ggt gtt acc gca gag ttc atg gaa gct ctg 1104
Gly Met Lys Ser His Pro Gly Val Thr Ala Glu Phe Met Glu Ala Leu
        355                 360                 365

cgc gat gtc aac gtg aac atc gaa ttg att tcc acc tct gag att cgt 1152
Arg Asp Val Asn Val Asn Ile Glu Leu Ile Ser Thr Ser Glu Ile Arg
    370                 375                 380

att tcc gtg ctg atc cgt gaa gat gat ctg gat gct gct gca cgt gca 1200
Ile Ser Val Leu Ile Arg Glu Asp Asp Leu Asp Ala Ala Ala Arg Ala
385                 390                 395                 400

ttg cat gag cag ttc cag ctg ggc ggc gaa gac gaa gcc gtc gtt tat 1248
Leu His Glu Gln Phe Gln Leu Gly Gly Glu Asp Glu Ala Val Val Tyr
                405                 410                 415

gca ggc acc gga cgc taa 1266
Ala Gly Thr Gly Arg
            420

<210> 6
<211> 421

<212>  PRT
<213>  Corynebacterium glutamicum ATCC13032

<400>  6

Met Ala Leu Val Val Gln Lys Tyr Gly Gly Ser Ser Leu Glu Ser Ala
1               5                   10                  15

Glu Arg Ile Arg Asn Val Ala Glu Arg Ile Val Ala Thr Lys Lys Ala
            20                  25                  30

Gly Asn Asp Val Val Val Val Cys Ser Ala Met Gly Asp Thr Thr Asp
        35                  40                  45

Glu Leu Leu Glu Leu Ala Ala Ala Val Asn Pro Val Pro Pro Ala Arg
    50                  55                  60

Glu Met Asp Met Leu Leu Thr Ala Gly Glu Arg Ile Ser Asn Ala Leu
65                  70                  75                  80

Val Ala Met Ala Ile Glu Ser Leu Gly Ala Glu Ala Gln Ser Phe Thr
                85                  90                  95

Gly Ser Gln Ala Gly Val Leu Thr Thr Glu Arg His Gly Asn Ala Arg
            100                 105                 110

Ile Val Asp Val Thr Pro Gly Arg Val Arg Glu Ala Leu Asp Glu Gly
        115                 120                 125

Lys Ile Cys Ile Val Ala Gly Phe Gln Gly Val Asn Lys Glu Thr Arg
    130                 135                 140

Asp Val Thr Thr Leu Gly Arg Gly Gly Ser Asp Thr Thr Ala Val Ala
145                 150                 155                 160

Leu Ala Ala Ala Leu Asn Ala Asp Val Cys Glu Ile Tyr Ser Asp Val
                165                 170                 175

Asp Gly Val Tyr Thr Ala Asp Pro Arg Ile Val Pro Asn Ala Gln Lys
            180                 185                 190

Leu Glu Lys Leu Ser Phe Glu Glu Met Leu Glu Leu Ala Ala Val Gly
        195                 200                 205

Ser Lys Ile Leu Val Leu Arg Ser Val Glu Tyr Ala Arg Ala Phe Asn
    210                 215                 220

Val Pro Leu Arg Val Arg Ser Ser Tyr Ser Asn Asp Pro Gly Thr Leu
225                 230                 235                 240

Ile Ala Gly Ser Met Glu Asp Ile Pro Val Glu Glu Ala Val Leu Thr
                245                 250                 255

Gly Val Ala Thr Asp Lys Ser Glu Ala Lys Val Thr Val Leu Gly Ile
            260                 265                 270

Ser Asp Lys Pro Gly Glu Ala Ala Lys Val Phe Arg Ala Leu Ala Asp
        275                 280                 285

Ala Glu Ile Asn Ile Asp Met Val Leu Gln Asn Val Ser Ser Val Glu
    290                 295                 300

Asp Gly Thr Thr Asp Ile Thr Phe Thr Cys Pro Arg Ser Asp Gly Arg
305                 310                 315                 320

Arg Ala Met Glu Ile Leu Lys Lys Leu Gln Val Gln Gly Asn Trp Thr
                325                 330                 335

Asn Val Leu Tyr Asp Asp Gln Val Gly Lys Val Ser Leu Val Gly Ala
            340                 345                 350

Gly Met Lys Ser His Pro Gly Val Thr Ala Glu Phe Met Glu Ala Leu
        355                 360                 365

Arg Asp Val Asn Val Asn Ile Glu Leu Ile Ser Thr Ser Glu Ile Arg
    370                 375                 380

Ile Ser Val Leu Ile Arg Glu Asp Asp Leu Asp Ala Ala Ala Arg Ala
385                 390                 395                 400

Leu His Glu Gln Phe Gln Leu Gly Gly Glu Asp Glu Ala Val Val Tyr
                405                 410                 415

Ala Gly Thr Gly Arg
            420

<210>  7
<211>  1603
<212>  DNA
<213>  Corynebacterium glutamicum

<220>
<221>  misc_feature
<222>  (1)..(801)
<223>  sequence upstream of the site of mutation (5'-flanking sequence)

<220>
<221>  CDS

<222>  (120)..(1601)

<220>
<221>  misc_feature
<222>  (801)..(803)
<223>  gac codon for aspartic acid at position 228

<220>
<221>  mutation
<222>  (802)..(802)
<223>  nucleobase adenine

<220>
<221>  misc_feature
<222>  (803)..(1603)
<223>  sequence downstream of the site of mutation (3'flanking sequence)

<220>
<221>  misc_feature
<222>  (1347)..(1349)
<223>  ttt codon for phenyalanine at position 410

<220>
<221>  misc_feature
<222>  (1349)..(1349)
<223>  nucleobase thymine

<400>  7
cccacgcgtc agtcaaaaat atggccaaca cttgcattcg ggtgctggcg atcatttatg      60

agatgacgcc ttgtgttggt gttcggcaga gaactcgcgg agataaaagg aagttgaac      119

atg tca gat tcc ccg aag aac gca ccg agg att acc gat gag gca gat      167
Met Ser Asp Ser Pro Lys Asn Ala Pro Arg Ile Thr Asp Glu Ala Asp
1               5                   10                  15

gta gtt ctc att ggt gcc ggt atc atg agc tcc acg ctg ggt gca atg      215
Val Val Leu Ile Gly Ala Gly Ile Met Ser Ser Thr Leu Gly Ala Met
            20                  25                  30

ctg cgt cag ctg gag cca agc tgg act cag atc gtc ttc gag cgt ttg      263
Leu Arg Gln Leu Glu Pro Ser Trp Thr Gln Ile Val Phe Glu Arg Leu
        35                  40                  45

gat gga ccg gca caa gag tcg tcc tcc ccg tgg aac aat gca gga acc      311
Asp Gly Pro Ala Gln Glu Ser Ser Ser Pro Trp Asn Asn Ala Gly Thr
    50                  55                  60

ggc cac tct gct cta tgc gag ctg aac tac acc cca gag gtt aag ggc      359
Gly His Ser Ala Leu Cys Glu Leu Asn Tyr Thr Pro Glu Val Lys Gly
65                  70                  75                  80

aag gtt gaa att gcc aag gct gta gga atc aac gag aag ttc cag gtt      407
Lys Val Glu Ile Ala Lys Ala Val Gly Ile Asn Glu Lys Phe Gln Val
                85                  90                  95

tcc cgt cag ttc tgg tct cac ctc gtt gaa gag gga gtg ctg tct gat      455
Ser Arg Gln Phe Trp Ser His Leu Val Glu Glu Gly Val Leu Ser Asp
            100                 105                 110

cct aag gaa ttc atc aac cct gtt cct cac gta tct ttc ggc cag ggc      503
Pro Lys Glu Phe Ile Asn Pro Val Pro His Val Ser Phe Gly Gln Gly
        115                 120                 125

gca gat cag gtt gca tac atc aag gct cgc tac gaa gct ttg aag gat     551
Ala Asp Gln Val Ala Tyr Ile Lys Ala Arg Tyr Glu Ala Leu Lys Asp
    130                 135                 140

cac cca ctc ttc cag ggc atg acc tac gct gac gat gaa gct acc ttc     599
His Pro Leu Phe Gln Gly Met Thr Tyr Ala Asp Asp Glu Ala Thr Phe
145                 150                 155                 160

acc gag aag ctg cct ttg atg gca aag ggc cgt gac ttc tct gat cca     647
Thr Glu Lys Leu Pro Leu Met Ala Lys Gly Arg Asp Phe Ser Asp Pro
                165                 170                 175

gta gca atc tct tgg atc gat gaa ggc acc gac atc aac tac ggt gct     695
Val Ala Ile Ser Trp Ile Asp Glu Gly Thr Asp Ile Asn Tyr Gly Ala
            180                 185                 190

cag acc aag cag tac ctg gat gca gct gaa gtt gaa ggc act gaa atc     743
Gln Thr Lys Gln Tyr Leu Asp Ala Ala Glu Val Glu Gly Thr Glu Ile
        195                 200                 205

cgc tat ggc cac gaa gtc aag agc atc aag gct gat ggc gca aag tgg     791
Arg Tyr Gly His Glu Val Lys Ser Ile Lys Ala Asp Gly Ala Lys Trp
    210                 215                 220

atc gtg acc gac aag aac gta cac act ggc gac acc aag acc atc aag     839
Ile Val Thr Asp Lys Asn Val His Thr Gly Asp Thr Lys Thr Ile Lys
225                 230                 235                 240

gca aac ttc gtg ttc gtc ggc gca ggc gga tac gca ctg gat ctg ctt     887
Ala Asn Phe Val Phe Val Gly Ala Gly Gly Tyr Ala Leu Asp Leu Leu
                245                 250                 255

cgc agc gca ggc atc cca cag gtc aag ggc ttc gct gga ttc cca gta     935
Arg Ser Ala Gly Ile Pro Gln Val Lys Gly Phe Ala Gly Phe Pro Val
            260                 265                 270

tcc ggc ctg tgg ctt cgt tgc acc aac gag gaa ctg atc gag cag cac     983
Ser Gly Leu Trp Leu Arg Cys Thr Asn Glu Glu Leu Ile Glu Gln His
        275                 280                 285

gca gcc aag gta tat ggc aag gca tct gtt ggc gct cct cca atg tct     1031
Ala Ala Lys Val Tyr Gly Lys Ala Ser Val Gly Ala Pro Pro Met Ser
    290                 295                 300

gtt cct cac ctt gac acc cgc gtt atc gag ggt gaa aag ggt ctg ctc     1079
Val Pro His Leu Asp Thr Arg Val Ile Glu Gly Glu Lys Gly Leu Leu
305                 310                 315                 320

ttt gga cct tac ggt ggc tgg acc cct aag ttc ttg aag gaa ggc tcc     1127
Phe Gly Pro Tyr Gly Gly Trp Thr Pro Lys Phe Leu Lys Glu Gly Ser
                325                 330                 335

tac ctg gac ctg ttc aag tcc atc cgc cca gac aac att cct tcc tac     1175
Tyr Leu Asp Leu Phe Lys Ser Ile Arg Pro Asp Asn Ile Pro Ser Tyr
            340                 345                 350

ctt ggc gtt gct gct cag gaa ttt gat ctg acc aag tac ctt gtc act     1223
Leu Gly Val Ala Ala Gln Glu Phe Asp Leu Thr Lys Tyr Leu Val Thr
        355                 360                 365

gaa gtt ctc aag gac cag gac aag cgt atg gat gct ctt cgc gag tac     1271
Glu Val Leu Lys Asp Gln Asp Lys Arg Met Asp Ala Leu Arg Glu Tyr

370                 375                 380

atg cca gag gca caa aac ggc gat tgg gag acc atc gtt gcc gga cag        1319
Met Pro Glu Ala Gln Asn Gly Asp Trp Glu Thr Ile Val Ala Gly Gln
385                 390                 395                 400

cgt gtt cag gtt att aag cct gca gga ttt cct aag ttc ggt tcc ctg        1367
Arg Val Gln Val Ile Lys Pro Ala Gly Phe Pro Lys Phe Gly Ser Leu
                405                 410                 415

gaa ttc ggc acc acc ttg atc aac aac tcc gaa ggc acc atc gcc gga        1415
Glu Phe Gly Thr Thr Leu Ile Asn Asn Ser Glu Gly Thr Ile Ala Gly
            420                 425                 430

ttg ctc ggt gct tcc cct gga gca tcc atc gca cct tcc gca atg atc        1463
Leu Leu Gly Ala Ser Pro Gly Ala Ser Ile Ala Pro Ser Ala Met Ile
        435                 440                 445

gag ctg ctt gag cgt tgc ttc ggt gac cgc atg atc gag tgg ggc gac        1511
Glu Leu Leu Glu Arg Cys Phe Gly Asp Arg Met Ile Glu Trp Gly Asp
    450                 455                 460

aag ctg aag gac atg atc cct tcc tac ggc aag aag ctt gct tcc gag        1559
Lys Leu Lys Asp Met Ile Pro Ser Tyr Gly Lys Lys Leu Ala Ser Glu
465                 470                 475                 480

cca gca ctg ttt gag cag cag tgg gca cgc acc cag aag acc ct             1603
Pro Ala Leu Phe Glu Gln Gln Trp Ala Arg Thr Gln Lys Thr
                485                 490


<210>  8
<211>  494
<212>  PRT
<213>  Corynebacterium glutamicum

<400>  8

Met Ser Asp Ser Pro Lys Asn Ala Pro Arg Ile Thr Asp Glu Ala Asp
1               5                   10                  15


Val Val Leu Ile Gly Ala Gly Ile Met Ser Ser Thr Leu Gly Ala Met
            20                  25                  30


Leu Arg Gln Leu Glu Pro Ser Trp Thr Gln Ile Val Phe Glu Arg Leu
        35                  40                  45


Asp Gly Pro Ala Gln Glu Ser Ser Ser Pro Trp Asn Asn Ala Gly Thr
    50                  55                  60


Gly His Ser Ala Leu Cys Glu Leu Asn Tyr Thr Pro Glu Val Lys Gly
65                  70                  75                  80


Lys Val Glu Ile Ala Lys Ala Val Gly Ile Asn Glu Lys Phe Gln Val
                85                  90                  95


Ser Arg Gln Phe Trp Ser His Leu Val Glu Glu Gly Val Leu Ser Asp

            100                 105                 110

Pro Lys Glu Phe Ile Asn Pro Val Pro His Val Ser Phe Gly Gln Gly
        115                 120                 125

Ala Asp Gln Val Ala Tyr Ile Lys Ala Arg Tyr Glu Ala Leu Lys Asp
    130                 135                 140

His Pro Leu Phe Gln Gly Met Thr Tyr Ala Asp Asp Glu Ala Thr Phe
145                 150                 155                 160

Thr Glu Lys Leu Pro Leu Met Ala Lys Gly Arg Asp Phe Ser Asp Pro
                165                 170                 175

Val Ala Ile Ser Trp Ile Asp Glu Gly Thr Asp Ile Asn Tyr Gly Ala
            180                 185                 190

Gln Thr Lys Gln Tyr Leu Asp Ala Ala Glu Val Glu Gly Thr Glu Ile
        195                 200                 205

Arg Tyr Gly His Glu Val Lys Ser Ile Lys Ala Asp Gly Ala Lys Trp
    210                 215                 220

Ile Val Thr Asp Lys Asn Val His Thr Gly Asp Thr Lys Thr Ile Lys
225                 230                 235                 240

Ala Asn Phe Val Phe Val Gly Ala Gly Gly Tyr Ala Leu Asp Leu Leu
                245                 250                 255

Arg Ser Ala Gly Ile Pro Gln Val Lys Gly Phe Ala Gly Phe Pro Val
            260                 265                 270

Ser Gly Leu Trp Leu Arg Cys Thr Asn Glu Glu Leu Ile Glu Gln His
        275                 280                 285

Ala Ala Lys Val Tyr Gly Lys Ala Ser Val Gly Ala Pro Pro Met Ser
    290                 295                 300

Val Pro His Leu Asp Thr Arg Val Ile Glu Gly Glu Lys Gly Leu Leu
305                 310                 315                 320

Phe Gly Pro Tyr Gly Gly Trp Thr Pro Lys Phe Leu Lys Glu Gly Ser
                325                 330                 335

Tyr Leu Asp Leu Phe Lys Ser Ile Arg Pro Asp Asn Ile Pro Ser Tyr
            340                 345                 350

Leu Gly Val Ala Ala Gln Glu Phe Asp Leu Thr Lys Tyr Leu Val Thr
        355                 360                 365

Glu Val Leu Lys Asp Gln Asp Lys Arg Met Asp Ala Leu Arg Glu Tyr
    370                 375                 380

Met Pro Glu Ala Gln Asn Gly Asp Trp Glu Thr Ile Val Ala Gly Gln
385                 390                 395                 400

Arg Val Gln Val Ile Lys Pro Ala Gly Phe Pro Lys Phe Gly Ser Leu
                405                 410                 415

Glu Phe Gly Thr Thr Leu Ile Asn Asn Ser Glu Gly Thr Ile Ala Gly
            420                 425                 430

Leu Leu Gly Ala Ser Pro Gly Ala Ser Ile Ala Pro Ser Ala Met Ile
        435                 440                 445

Glu Leu Leu Glu Arg Cys Phe Gly Asp Arg Met Ile Glu Trp Gly Asp
    450                 455                 460

Lys Leu Lys Asp Met Ile Pro Ser Tyr Gly Lys Lys Leu Ala Ser Glu
465                 470                 475                 480

Pro Ala Leu Phe Glu Gln Gln Trp Ala Arg Thr Gln Lys Thr
                485                 490
201700371           1